# EUROPEAN PATENT SPECIFICATION

(11) **EP 0 963 550 B1**
(45) Date of publication and mention of the grant of the patent: **09.06.2010**
(21) Application number: 98905449.9
(22) Date of filing: 26.02.1998
(51) Int. Cl.: G01N 27/333

(54) **USE OF AN ION-SELECTIVE ELECTRODE AND METHOD FOR SELECTIVE DETERMINATION OF CARBON DIOXIDE IN BODY FLUIDS**
VERWENDUNG EINER IONENSELEKTIVE ELEKTRODE UND VERFAHREN ZUR BESTIMMUNG VON KOHLENSÄURE IN KÖRPERFLÜSSIGKEITEN
EMPLOI D'UNE ELECTRODE SELECTIVE D'IONS ET PROCEDE DE DETERMINATION SELECTIVE DU DIOXYDE DE CARBONE DANS DES FLUIDES PHYSIOLOGIQUES

(30) Priority: 26.02.1997 FI 970816
(43) Date of publication of application: 15.12.1999
(73) Proprietor: Konelab Corporation, 02320 Espoo (FI)
(72) Inventor: LEWENSTAM, Andrzej, FIN-00330 Helsinki (FI); BOBACKA, Johan, FIN-20810 Abo (FI)
(74) Representative: Sundman, Patrik Christoffer
(86) International application number: PCT/FI1998/000179
(87) International publication number: WO 1998/038503

(56) References cited:
- EP-A- 0 603 742
- DD-A- 240 265
- US-A- 3 598 713
- US-A- 4 272 328
- DATABASE WPI Week 199242, Derwent Publications Ltd., London, GB; Class A89, AN 1992-347284 & SU 1 672 339 A1 (UNIV MOSC LOMONOSOV) 23 August 1991

## Description

### Background of the Invention

### Field of the Invention

The present invention relates to the determination of analytes in aqueous samples. In particular, the invention concerns use of an ion-selective eletrode and a method for determining the total concentration of carbon dioxide and other analytes in body fluids, e.g. blood, plasma or serum.

### Description of Related Art

The acid-base and the electrolyte balance in the human body is regulated by pulmonary and renal mechanisms. Bicarbonate is the main ion connecting these two regulative mechanisms. A dysfunction in either of the two mechanisms is generally manifested by changes in the bicarbonate concentration accompanied by concentration changes of other anions and pH.

The actual concentration of bicarbonate is interdependent on the total concentration of all other forms in which CO₂ exists in blood, namely dissolved carbon dioxide, carbonic acid, carbonates, carbamates, and on pH [Tietz Fundamentals of Clinical Chemistry, C.A.Burtis and E.R.Ashwood, eds, fourth edition, W.B.Saunders, Co., Philadelphia, p.525]. For this reason the measurement of total CO₂ belongs to the group of highest importance in the clinical laboratory.

The chemical conditions of blood are such that the value of total CO₂ can be easily calculated if only pH and the concentration of one form of CO₂ are measured. This is why carbon dioxide sensors able to measure dissolved CO₂ or carbonate ion-selective electrodes have been used in the prior art (cf. U.S. Pat. No. 3,723,281).

The main disadvantage of the gas sensor is its mechanically complicated structure and a need for calibration with gases.

The carbonate ion-selective electrodes described so far are lacking sensitivity and selectivity. The insufficient sensitivity requires inconvenient adjustment (i.e. increase) of the pH of the sample by addition of an alkaline buffer prior to the measurement, or introduction of sophisticated layers alkalizing the electrode surface (U.S. Pat. No. 4,272,328). Thus, electrodes in serial measurements cannot be applied.

The attempts made to improving the sensitivity have adversely affected the selectivity, especially for frequently used medicines, such as the aspirins (salicylates). This has, in turn, resulted in a need for introducing an interferent-removing zone (cf, U.S. Pat. No. 4,303,408 ), which adds to the mechanical complexity of the electrode, though still did not allowing for salicylate-free measurement.

Furthermore, interference from substances such as proteins and surfactants can be regarded as a general problem associated with the determination of electrolytes (Na⁺, K⁺, Cl⁻, Ca²⁺, Mg²⁺, Li⁺, H⁺) with polymeric (e.g. PVC) membrane electrodes, cf. Oesch, D. Ammann and W. Simon, "Ion-selective membrane electrodes for clinical use" Clinical Chemistry, Vol. 32, No. 8, 1986, pp. 1448-1459*,* and C. Espadus-Torre, E. Bakker, S. Barker and M.E. Meyerhoff, "influence of nonionic surfactants on the potentiometric response of hydrogen ion-selective polymeric membrane electrodes", Analytical Chemistry, Vol. 68, No. 9, May 1, 1996, pp. 1623-1631*)*.

SU 1672339 discloses an ion-selective electrode device, for example, for hydrometallurgical production of gold. The electrode comprises an internal reference electrode, and a liquid phase ion-selective membrane in intimate contact with an outer cellophane film.

US 3598713 discloses an electrode for measuring the concentration of potassium ions in samples. The electrode comprises a hydrophobic outer layer between an ion-selective membrane and the sample.

DD 240265 discloses an electrode device utilizing a particle filter made of glass.

EP 0603742 discloses describes an ion selective electrode for carbon dioxide analyses. The electrode comprises an ion selective layer which is in contact with an asymmetric membrane. The ion selective layer comprises a polymer. The asymmetric membrane may comprise cellulose triacetate layer in order to form OH-groups through hydrolysis on the surface of the electrode.

### Summary of the Invention

The present invention aims at eliminating the problems relating to the prior art and it is an object of the invention to provide a method utilizing an ion-selective electrode, which makes it possible directly to determine the total carbon dioxide concentration of body fluids and which makes it possible to decrease the interference from other substances such as proteins and surfactants when measuring other ions such as Na⁺, K⁺, Cl⁻, Ca²⁺, Mg²⁺, Li⁺ and H⁺ with ion-selective electrodes.

In particular, the invention aims at providing a membrane combination suitable for determining carbonates present in body fluids containing salicylate ions.

It is also an object of the invention to provide a novel method for direct determination of total CO₂ of body fluids without changing the pH of the sample.

These and other objects, together with the advantages thereof over known ion-selective electrodes and methods for using such electrodes and sensors, which shall become apparent from the specification which follows, are accomplished by the invention as hereinafter described and claimed. More specifically, the method is characterized in claim 1 and the use in claim 14.

In connection with the present invention, it has been found that it is possible to provide interferent-free, in particular salicylate-free, determination of carbonates by an ion-selective electrode without pH adjustment if said electrode comprises a liquid-phase ion-selective membrane, which is intimately contacted with a discriminating membrane.
Although we do not wish to be bound to any specific theory, it appears that it is the interphase between the discriminating membrane and the liquid-phase ion-selective membrane which is mainly responsible for the discrimination of the ions rather than the discriminating membrane as such. However, for the purpose of the present invention, the term "discriminating membrane" is used for a hydrophilic membrane having small pores, which is permeable to the analyte but apparently (e.g. as a result of an interaction with the liquid ion-selective membrane) essentially impermeable to the interferent. Preferably the hydrophilic membrane has a medium pore size of less than 0.1 µm, in particular less than 0.01 µm.

Said "carbonate-selective membrane" is essentially hydrophobic and comprises a substance specifically binding the carbonate ions. If that substance is in itself a liquid, it can be used as such. Otherwise, the substance is dissolved in a solvent to provide a liquid-phase membrane. For interferent-free determination, the liquid phase should preferably be essentially free of dissolved polymers typically used in conventional carbonate selective membranes.

More specifically, the present ion-selective electrode comprises
- an internal reference electrode,
- a liquid-phase carbonate-selective membrane and
- a discriminating membrane comprising a hydrophilic membrane having a medium pore size of less than 0.1 µm, in particular less than about 0.01 µm,
   said liquid-phase carbonate-selective membrane being placed in intimate contact with said discriminating membrane.
   The novel membrane structure for use in the invention comprises in combination
- a hydrophobic liquid-phase carbonate selective membrane which is essentially free of polymers dispersed in the liquid phase, and
- a discriminating membrane placed in intimate contact with said carbonate-selective membrane,
   said discriminating membrane being permeable to carbonate and essentially non-permeable to the interferent.

The present method for determining the total carbon dioxide concentration or the concentration (activity) of other ions in body fluids comprises using two or, preferably, three electrodes in serial arrangement. According to the present method, a sample of the body fluid is taken and a first portion thereof is contacted with a ion-selective electrode of the above-described structure, a second portion of the sample is contacted with a conventional (external) reference electrode to complete the electrical circuit and the voltage difference between the two electrodes is determined. From the signal obtained the carbon dioxide concentration or the concentration (activity) of other ions in the sample is calculated.

Preferably, a third portion of the sample is used for determination of the pH of the sample, which data is utilized for calculation of the carbon dioxide concentration. In direct measurements, pH is measured simultaneously with the carbonate by a third electrode or sensor placed in serial arrangement with the other two electrodes. The measurement of pH is not a prerequisite for utilizing this invention for the determination of other ions such as magnesium ions.

### Brief Description of the Drawings

Figure 1 is a cross-sectional view of an ion-selective electrode according to the invention;
Figure 2 a simplified representation of the structure of the present ion-selective electrode; and
Figure 3 depicts in graphical form the total carbon dioxide concentration of blood samples tested with the present ion-selective electrode.

### Detailed Description of the Invention

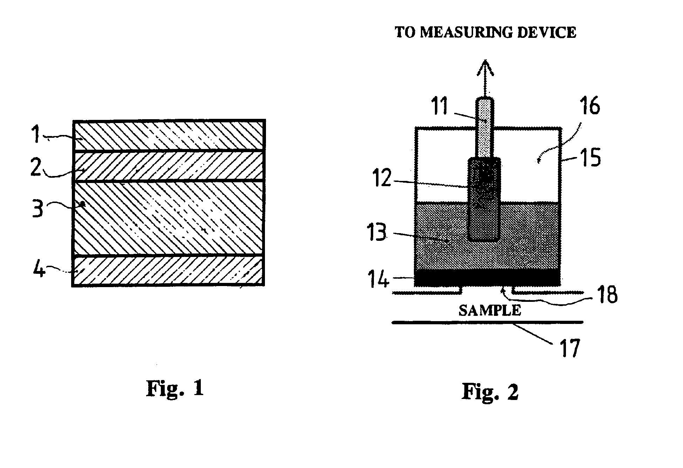

Figure 1 gives a rough overview of an ion-selective electrode according to the invention. Reference numerals 1 and 2 refer to the internal reference electrode which can consist on different layers 1 and 2, as will be explained below in more detail. Numeral 3 indicates the liquid ion-selective membrane and numeral 4 the discriminating membrane.

As explained above, in connection with the present invention it has been found that certain combinations of positions of the membrane of the ion-selective electrode and certain constructions of the electrode containing the same membranes exhibit high sensitivity and high selectivity for carbonate ions over interferents, in particular over interfering salicylates.

The basic concept of the invention is to use a discriminating membrane to exclude potential interferents from the carbonate-selective membrane. Based on this concept, various potentially useful discriminating membranes were tested. Surprisingly only hydrophilic membranes having small medium pore sizes have been found to be effective. In particular, a hydrophilic membrane comprising fibers based on cellulose, regenerated cellulose or cellulose derivatives, has been found effectively to exclude salicylates due to a combined pore dimension-charge effect.

The thickness of said disciminating membrane should be less than about 200 µm, in particular less than 100 µm. Preferably, the discriminating membrane comprises a membrane made from viscose fibers and having a medium pore size of less than about 0.1 µm, in particular less than about 0.01 µm. The discriminating membrane used in the examples below comprises a cellophane film (a film made from regenerated cellulose fibers) having a thickness of less than about 50 µm and a medium pore size of less than about 0.005 µm.

The discriminating membrane impedes the entry of interfering ions while allowing for the intimate contact with the carbonate ions.

However, it has turned out that the discriminating properties of the above-described membrane are obtained only if the ion-selective membrane used is a liquid carbonate-selective membrane, not a conventional plastic-based one. Furthermore, the ion-selective membrane has to be placed in intimate contact with the discriminating membrane. Thus, the use of a liquid ion-selective membrane in close physical and chemical contact with the discriminating, preferably hydrophilic, membrane is a characteristic feature of the invention described herein.

The ion-selective membrane should be essentially free of polymers dissolved in the liquid phase. Typically, present state-of-the art carbon-selective membranes comprise poly(vinyl chloride) dissolved in a solvent to provide an essentially solid-phase (dry) membrane. In contrast, the present membrane is a liquid at room temperature and it comprises a substance specifically binding carbonate ions and a solvent thereof. In addition to these components, the membrane can contain mediating substances and impeding substances.

By "essentially free of polymers" it is meant that there should be no or only minute amounts of, e.g. PVC and similar polymers dissolved in the liquid phase, such that they do not interfer with the operation of the combination formed by the carbonate-selective membrane and the discriminating membrane. Generally, the amount of these polymers should be less than about 2-3 wt-%, preferably less than 1 wt-%, in particular less than 0.1 wt-%. Some amounts of dispersed polymers can be allowed in the liquid phase.

Thus, the ion-selective membrane generally comprises
- about 5 to 50 mol-% of an ionophore,
- 0 to about 10 mol-% of a mediating substance,
- 0 to about 10 mol-% of a impeding substance, and
- about 30 to 95 mol-% of a solvent.

Said substance specifically binding carbonate ions is preferably an ionophore is selected from the group consisting of 1-trifluoroacetophenones, such as 4-butyl-α,α,α-trifluoroacetophenone and 4-hexadecyl-α,α,α-trifluoroacetophenone, trifluoroacetophenone, 1-(dodecylsulfonyl)-4-trifluoroacetylbenzene, heptyl-4-trifluoroacetylbenzoate, and N-dodecyl-4-trifluoro-acetylacetanilide. Of the above-mentioned ionophores, 4-butyl-α,α,α-trifluoroaceto-phenone is a liquid and can be used as such in the membrane without any solvent.

For the determination of anions, the mediating substance, if any, is preferably a quaternary ammonium salt, which can be selected from the group consisting of tridodecyl-methyl-ammonium chloride and tridodecylmethylammonium carbonate.

The impeding substance is also a quaternary ammonium salt, such as tetradodecylammonium tetrakis (4-chlorophenyl)borate.

The solvent of the ion-selective membrane should be capable of dissolving all of the components contained therein. Suitable solvents include the adipates, sebacates, and ethers and mixtures thereof. In particular, the solvent can be selected from the group consisting of bis(2-ethylhexyl)adipate, bis(2-ethylhexyl)sebacate and 2-nitrophenyl octyl ether.

Thus, a membrane structure comprising in combination a liquid carbonate selective membrane which is essentially free of polymers dispersed in the liquid phase, and a hydrophilic membrane placed in intimate contact with said carbonate-selective membrane, said discriminating membrane being permeable to the analyte and essentially non-permeable to the interferent, can be used in connection with any conventional ion-selective electrode structure. As an example of alternative analytes, chloride can be mentioned. Typical interferents are exemplified by proteins, ascorbic acid, salicylate, p-aminosalicylate, gentisic acid, thiocyanate and tetrahexylammonium chloride. As another example of alternative analytes, magnesium can be mentioned. Typical interferents are exemplified by proteins and surfactants.

The present invention can be used to analyze aqueous liquids, such as water containing analytes. Preferably it is used for analyzing body fluids, such as blood, serum or plasma.

The carbonate-selective membrane compositions and electrodes containing same are prepared according to the procedures typically applied for the preparation of any traditional ion-selective electrode. However, the electrode body is specially designed to make possible incorporation of all electrode elements.

Figure 2 shows the simplified layout of a carbonate-selective electrode according to the present invention.

The present an carbonate-selective electrode for determination of total CO₂ in physiological fluids comprises a housing or support 15 having therein and thereon, in sequence: an internal reference electrode 11, 12, an ion-selective membrane 13 and a discriminating membrane 14. Depending on the mechanical strengthness of the discriminating membrane 14 it can be self-supported or arranged on, e.g., a wire gause or another permeable support means. A sample channel 17 is arranged next to the discriminating membrane and provided with an opening 18 facing said membrane so as to contact a sample flowing through the channel with the membrane. The internal reference electrode is provided with contact means for coupling with a measuring device.

In the present embodiment, the space 16 above the carbonate-selective membrane is filled with gas, such as air. It can also be evacuated. Further, space 16 can also be eliminated by lowering the roof of the space to contact with the liquid surface of the ion selective membrane.

As will be seen from the figure, it is an advantageous feature of the invention that the internal reference electrode 11, 12 can be directly immersed into the ion-selective membrane 13. However, connection of the internal electrode via an intermediate charge-mediating liquid or solid layer is also within the spirit and scope of the invention, as apparent to person skilled in the art. This liquid or solid layer is then placed in space 16.

The internal reference electrode can consist of any known electrodes, such as metals 11 and metal halides 12, and metals and electroactive polymers. Redox polymers and conductive polymers are examples of the electroactive polymers. Particularly preferred combinations consist of noble metals and nobel metal halides, such as silver and silver chloride. The internal reference electrode can also consist of an internal ion-selective electrode.

According to a preferred embodiment, the metal halide portion of the electrode is immersed into the carbonate-selective membrane. The metal halide can be coated with a polymer layer to decrease the dissolution of the metal ions from the metal halide.

An external reference electrode and a pH electrode (not shown) can be inserted into the sample channel to provide for simultaneous determination of a reference voltage and pH.

The above-described apparatus is used as follows:
A sample of the body fluid of interest is taken and conducted through the sample channel The first portion of the sample is brought into contact with the discriminating membrane at the opening. Carbonate ions of the sample will penetrate through the discriminating membraned and reach the liquid carbonate-selective membrane, where they will interact with the ionophore.

The carbonate-binding ionophore molecule will enter into electrical equilibrium with the internal electrode, thus creating a first potential. The discriminating membrane in combination with the ionophore of the carbonate-selective membrane will prevent the interferents from interacting with the components of the carbonate-selective membrane and therefore the interferents will not influence the potential of the ion-selective electrode.

The sample (or the second and third portions thereof) is also in contact with the external reference electrode and pH electrode. The reference electrode can be of any known structure and is in electrical contact with the sample either directly or through a bridge. The reference electrode establishes a second potential in equilibrium with the sample and by measuring the voltage difference between the ion-selective electrode and the external reference electrode, respectively, a voltage difference corresponding to the concentration of carbonate ions in the sample will be obtained. By comparing said voltage with reference voltages of predetermined carbonate concentrations it is possible to determine the concentration soluble carbonates. By measuring the pH of the sample, it is possible to convert this value into the total carbon dioxide in the sample.

It should be pointed out that although various "portions" of the sample are referred to above, said portions all belong to the same sample plug in the channel, they form the same sample and are in electrical contact with each other via the ions of the sample (there is formed a closed circuit between the ion-selective electrode and the external reference electrode).

The following non-limiting example illustrates the invention in more detail.

### Example

An ion-selective electrode for CO₂ determination was manufactured as follows:

| | | |
|---|---|---|
| Discriminating membrane: | Cellophane (supplied by | 35 g/m² |
| | Wistom-Chemtex, Poland) | ca 20 µm |
| | (Ion-discriminating membrane) | |
| | | |
| Ion-selective membrane: | 4-(n-hexadecyl)-α,α,α,-trifluoro- | 25 mol % |
| | acetophenone | |
| | (Ionophore) | |
| | | |
| | Tridodecylmethylammonium | 2.5 mol % |
| | carbonate | |
| | (Mediating substance) | |
| | | |
| | Tetradodecylammonium tetrakis | 2.5 mol % |
| | (4-chlorophenyl)borate | |
| | (Impeding substance) | |
| | | |
| | Bis(2-ethylhexyl) adipate | 70 mol % |
| | (Solvent) | |
| | | |
| Internal electrode: | Silver/silver chloride | |

Using the above ion-selective electrode, electrical potential differences of blood serum samples having various carbon dioxide concentrations were studied in the presence and absence of salicylate ions.

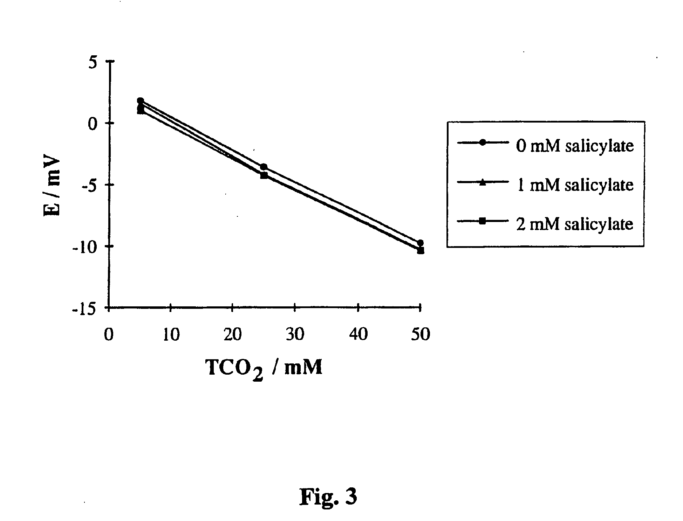

Figure 3 depicts the electrical potential of the serum sample depending on TCO₂ for three different salicylate concentrations (0, 1 and 2 mM salicylate).

As is apparent from the Figure, for practical purposes, the electrical potential is independent of the salicylate concentration, which means that the present ion-selective electrode provides for interferent-free determination of carbonate.

The invention has been described in detail with particular reference to preferred embodiments thereof, but it will be understood that variations and modifications can be effected within the scope of the invention as defined by the claims.

## Claims

1. A method of determining the total concentration of carbon dioxide in body fluids, comprising the steps of
- taking a sample of the body fluid,
- contacting a first portion of the sample with an ion-selective electrode including an internal reference electrode (1, 2; 11, 12), a liquid-phase carbonate-selective membrane (3; 13) and a discriminating membrane (4; 14) comprising a hydrophilic membrane having a medium pore size of less than 0.1 µm, said liquid-phase carbonate-selective membrane (3; 13) being placed in intimate contact with said discriminating membrane (4; 14),
- contacting a second portion of the sample, which is in contact with the first portion of the sample, with an external reference electrode,
- measuring the voltage difference between the external reference electrode and the ion-selective electrode corresponding to the concentration of carbonate ions in the sample and
- comparing said voltage with reference voltages of predetermined carbon dioxide concentrations in order to determine the concentration of carbon dioxide in the sample.

2. The method according to claim 1, wherein the pH of the sample is simultaneously measured.

3. The method according to claim 1 or 2, wherein the body fluid comprises blood, plasma or serum.

4. The method according to any of the preceding claims, wherein a discriminating membrane (4; 14) selected from the group consisting of membranes made from cellulose fibers, regenerated cellulose fibers, and fibers of cellulose derivatives is used.

5. The method according to any of the preceding claims, wherein a discriminating membrane (4; 14) having a thickness less than 200 µm is used.

6. The method according to any of the preceding claims, wherein a discriminating membrane (4; 14) comprising a membrane made from viscose fibers and having a medium pore size of less than about 0.01 µm is used.

7. The method according to claim 6, wherein a discriminating membrane (4; 14) comprising a cellophane film having a thickness of less than about 50 µm is used.

8. The method according to any of the preceding claims, wherein a carbonate-selective membrane (3; 13) essentially free of polymers dissolved in the liquid phase is used.

9. The method according to any of the preceding claims, wherein a carbonate-selective membrane (3; 13) comprising a substance specifically binding carbonate ions and a solvent is used.

10. The method according to any of the preceding claims, wherein a carbonate selective membrane (3; 13) comprising
- a substance specifically binding carbonate ions,
- a mediating substance, such as quaternary ammonium salt, preferably selected from the group consisting of tridodecyl-methyl-ammonium chloride and tridodecylmethylammonium carbonate,
- an impeding substance, such as quaternary ammonium salt, preferably tetradodecylammonium tetrakis (4-chlorophenyl)borate, and
- a solvent
is used.

11. The method according to claim 9 or 10, wherein said substance specifically binding carbonate ions is an ionophore, preferably selected from the group consisting of 4-butyl-α,α,α-trifluoroacetophenone, 4-hexadecyl-α,α,α-trifluoroacetophenone, 1-(dodecylsulfonyl)-4-trifluoroacetylbenzene, 4-heptyl-α,α,α-trifluoroacetylbenzoate, 4-N-dodecyl-α,α,α-trifluoroacetylacetanilide.

12. The method according to claim 9 or 10, wherein said solvent is adipate, a sebacates, an ether or a mixture thereof, preferably selected from the group consisting of bis(2-ethylhexyl)adipate, bis(2-ethylhexyl)sebacate and 2-nitrophenyl octyl ether.

13. The method according to any of the preceding claims, wherein an internal reference electrode (1, 2; 11, 12) at least partially immersed into the carbonate-selective membrane (3; 13) is used.

14. Use of an ion-selective electrode comprising
- an internal reference electrode (1, 2; 11, 12),
- a liquid-phase carbonate selective membrane (3; 13) and
- a discriminating membrane (4; 14) comprising a hydrophilic membrane having a medium pore size of less than 0.1 µm, said liquid-phase ion-selective membrane (3; 13) being placed in intimate contact with said discriminating membrane (4; 14)
for determination of carbon dioxide in body fluids.

15. The use according to claim 14, wherein said discriminating membrane (4; 14) is selected from the group consisting of membranes made from cellulose fibers, regenerated cellulose fibers, and fibers of cellulose derivatives.

16. The use according to claim 14 or 15, wherein the thickness of said discriminating membrane (4; 14) is less than 200 µm.

17. The use according to any of claims 14 to 16, wherein said discriminating membrane (4; 14) comprises a membrane made from viscose fibers and having a medium pore size of less than about 0.01 µm.

18. The use according to claim 17, wherein said discriminating membrane (4; 14) comprises a cellophane film having a thickness of less than about 50 µm.

19. The use according to any of claims 14 - 18, wherein said carbonate-selective membrane (3; 13) is essentially free of polymers dissolved in the liquid phase.

20. The use according to any of claims 14 - 19, wherein said carbonate-selective membrane (3; 13) comprises a substance specifically binding carbonate ions and a solvent.

21. The use according to any of claims 14 - 20, wherein said carbonate-selective membrane (3; 13) comprises a substance specifically binding carbonate ions, a mediating substance, an impeding substance and a solvent.

22. The use according to claim 20 or 21, wherein said substance specifically binding carbonate ions is an ionophore

23. The use according to claim 22, wherein said ionophore is selected from the group consisting of 4-butyl-α,α,α-trifluoroacetophenone, 4-hexadecyl-α,α,α-trifluoroacetophenone, 1-(dodecylsulfonyl)-trifluoroacetylbenzene, 4-heptyl-α,α,α-trifluoroacetylbenzoate, 4-N-dodecyl-α,α,α-trifluoroacetylacetanilide.

24. The use according to claim 21, wherein said mediating substance is a quaternary ammonium salt.

25. The use according to claim 24, wherein said mediating substance is a quaternary ammonium salt, selected from the group consisting of tridodecyl-methyl-ammonium chloride and tridodecylmethylammonium carbonate.

26. The use according to claim 21, wherein said impeding substance is a quaternary ammonium salt.

27. The use according to claim 26, wherein said quaternary ammonium salt is tetradodecylammonium tetrakis (4-chlorophenyl)borate.

28. The use according to claim 20 or 21, wherein said solvent is an adipate, a sebacates, an ether or a mixture thereof.

29. The use according to claim 28, wherein said solvent is selected from the group consisting of bis(2-ethylhexyl)adipate, bis(2-ethylhexyl)sebacate and 2-nitrophenyl octyl ether.

30. The use according to any of claims 14 - 29, wherein the internal reference electrode (1, 2; 11, 12) is at least partially immersed into the carbonate-selective membrane (3; 13).

## Patentansprüche

1. Verfahren zur Bestimmung der totalen Kohlendioxidkonzentration in Körperflüssigkeiten, umfassend die folgenden Schritte:
- Entnahme einer Probe der Körperflüssigkeit,
- Kontaktieren einer ersten Partie der Probe mit einer ionenselektiven Elektrode, die eine interne Referenzelektrode (1, 2; 11, 12), eine Flüssigphasen-karbonatselektive Membran (3; 13) und eine diskriminierende Membran (4; 14) mit einer hydrophilen Membran mit einer mittleren Porengröße von weniger als 0,1 µm aufweist, wobei die Flüssigphasenkarbonatselektive Membran (3; 13) in engem Kontakt mit der diskriminirenden Membran (4; 14) steht,
- Kontaktieren einer mit der ersten Partie der Probe in Verbindung stehenden zweiten Partie des Probe mit einer externen Referenzelektrode,
- Messung der Spannungsdifferenz zwischen der externen Referenzelektrode und der ionenselektiven Elektrode entsprechend der Konzentration der Karbonationen In der Probe und
- Vergleich der Spannung mit Referenzspannungen von vorbestimmten Kohlendioxidkonzentrationen zur Bestimmung der Konzentration von Kohlendioxid in der Probe.

2. Verfahren nach Anspruch 1, bei dem gleichzeitig der pH-Wert der Probe gemessen wird.

3. Verfahren nach Anspruch 1 oder 2, bei dem die Körperflüssigkeit Blut, Plasma oder Serum aufweist.

4. Verfahren nach einem der vorangehenden Ansprüche, bei dem eine diskriminierende Membran (4; 14) ausgewählt aus der Gruppe bestehend aus Membranen hergestellt aus Zellulosefasern, regenerierten Zellulosefasern und Fasern aus Zellulosederivaten verwendet wird.

5. Verfahren nach einem der vorangehenden Ansprüche, bei dem eine diskrimierende Membran (4; 14) mit einer Dicke von weniger als 200 µm verwendet wird.

6. Verfahren nach einem der vorangehenden Ansprüche bei dem eine diskriminierende Membran (4; 14) mit einer aus Viskosefasern hergestellten Membran mit einer mittleren Porengröße von weniger als etwa 0,01 µm verwendet wird.

7. Verfahren nach Anspruch 6, bei dem eine diskrimierende Membran (4; 14) mit einer Zellophanschicht mit einer Dicke von weniger als etwa 50 µm verwendet wird.

8. Verfahren nach einem der vorangehenden Ansprüche, bei dem eine karbonatselektive Membran (3; 13) verwendet wird, die im Wesentlichen frei von in der flüssigen Phase gelösten Polymeren ist.

9. Verfahren nach einem der vorangehenden Ansprüche, bei dem eine karbonatselektive Membran (3; 13) verwendet wird, die eine spezifisch Karbonationen bindende Substanz und ein Lösungsmittel aufweist.

10. Verfahren nach einem der vorangehenden Ansprüche, bei dem eine karbonatselektive Membran (3; 13) verwendet wird, die
- eine spezifisch Karbonationen bindende Substanz,
- eine vermittelnde Substanz, beispielsweise quaternäres Ammoniumsalz, vorzugsweise ausgewählt aus der Gruppe bestehend aus Tridodezylmethylammoniumchlorid und Tridodezylmethylammoniumkarbonat,
- eine hemmende Substanz, beispielsweise ein quaternäres Ammoniumsalz, vorzugsweise Tetradodezylammoniumtetrakis-(4-Chlorophenyl)-Borat und
- ein Lösungsmittel
aufweist.

11. Verfahren nach Anspruch 9 oder 10, bei dem die spezifisch Karbonationen bindende Substanz ein lonophor ist, vorzugsweise ausgewählt aus der Gruppe bestehend aus 4-Butyl-α,α,α-Trifluoroacetophenon, 4-Hexadezyl-α,α,α-Trifluoroacetophenon, 1-(Dodezylsulfonyl)-4-Trifluoroacetylbenzen, 4-Heptyl-α,α,α-Trifluoroacetylbenzoat, 4-N-Dodezyl-α,α,α-Trifluoroacetylacetanilid.

12. Verfahren nach Anspruch 9 oder 10, bei dem das Lösungsmittel ein Adipinat, ein Sebacinat, ein Äther oder eine Mischung daraus ist, vorzugsweise ausgewählt aus der Gruppe bestehend aus Bis-(2-Ethylhexyl)-Adipinat, Bis-(2-Ethylhexyl)-Sebacinat und 2-Nitrophenyl-Octyl-Äther.

13. Verfahren nach einem der vorangehenden Ansprüche, bei dem eine interne Referenzelektrode (1, 2; 11, 12), die zumindest teilweise in die karbonatselektive Membran (3; 13) eintaucht, verwendet wird.

14. Verwendung einer ionenselektiven Elektrode mit
- einer internen Referenzelektrode (1, 2; 11, 12),
- einer Flüssigphasen-karbonatselektiven Membran (3; 13) und
- einer diskriminierende Membran (4; 14) mit einer hydrophilen Membran mit einer mittleren Porengröße von weniger als 0,1 µm, wobei die Flüssigphasen-ionenselektive Membran (3; 13) in engem Kontakt mit der diskriminirenden Membran (4; 14) steht,
zur Bestimmung von Kohlendioxid in Kärperflüssigkeiten.

15. Verwendung nach Anspruch 14, bei der die diskriminierende Membran (4; 14) ausgewählt ist aus der Gruppe bestehend aus Membranen hergestellt aus Zellulosefasern, regenerierten Zellulosefasern und Fasern aus Zellulosederivaten.

16. Verwendung nach Anspruch 14 oder 15, bei der die diskrimierende Membran (4; 14) eine Dicke von weniger als 200 µm aufweist.

17. Verwendung nach einem der Ansprüche 14 bis 16, bei der die diskriminierende Membran (4; 14) eine aus Viskosefasern hergestellte Membran mit einer mittleren Porengröße von weniger als etwa 0,01 µm aufweist.

18. Verwendung nach Anspruch 17, bei der die diskrimierende Membran (4; 14) eine Zellophanschicht mit einer Dicke von weniger als etwa 50 µm aufweist.

19. Verwendung nach einem der Ansprüche 14 bis 18, bei der die karbonatselektive Membran (3; 13) im Wesentlichen frei von in der flüssigen Phase gelösten Polymeren ist.

20. Verwendung nach einem der Ansprüche 14 bis 19, bei der die karbonatselektive Membran (3; 13) eine spezifisch Karbonationen bindende Substanz und ein Lösungsmittel aufweist.

21. Verwendung nach einem der Ansprüche 14 bis 20, bei der die karbonatselektive Membran (3; 13) eine spezifisch Karbonationen bindende Substanz, eine vermittelnde Substanz, eine hemmende Substanz und ein Lösungsmittel aufweist.

22. Verwendung nach Anspruch 20 oder 21, bei der die spezifisch Karbonationen bindende Substanz ein Ionophor ist.

23. Verwendung nach Anspruch 22, bei der das Ionophor ausgewählt ist aus der Gruppe bestehend aus 4-Butyl-α,α,α-Trifluoroacetophenon, 4-Hexadezyl-α,α,α-Trifluoroacetophenon, 1-(Dodezylsulfonyl)-4-Trifluoroacetylbenzen, 4-Heptyl-α,α,α-Trifluoroacetylbenzoat, 4-N-Dodezyl-α,α,α-Trifluoroacetylacetanilid.

24. Verwendung nach Anspruch 21, bei der die vermittelnde Substanz ein quaternäres Ammoniumsalz ist.

25. Verwendung nach Anspruch 24, bei der die vermittelnde Substanz ein quaternäres Ammoniumsalz ist, ausgewählt aus der Gruppe bestehend aus Tridodezylmethylammoniumchlorid und Tridodezylmethylammoniumkarbonat.

26. Verwendung nach Anspruch 21, bei der die hemmende Substanz ein quaternäres Ammoniumsalz ist.

27. Verwendung nach Anspruch 26, bei der das quaternäre Ammoniumsalz Tetradodezylammoniumtetrakis-(4-Chlorophenyl)-Borat ist.

28. Verwendung nach Anspruch 20 oder 21, bei der das Lösungsmittel ein Adipinat, ein Sebacinat, ein Äther oder eine Mischung daraus ist.

29. Verwendung nach Anspruch 28, bei der das Lösungsmittel ausgewählt ist aus der Gruppe bestehend aus Bis-(2-Ethylhexyl)-Adipinat, Bis-(2-Ethylhexyl)-Sebacinat und 2-Nitrophenyl-Octyl-Äther.

30. Verwendung nach einem der Ansprüche 14 bis 29, bei der die interne Referenzelektrode (1, 2; 11, 12) zumindest teilweise in die karbonatselektive Membran (3; 13) eintaucht.

## Revendications

1. Procédé de détermination de la concentration totale en dioxyde de carbone dans des fluides corporels, comprenant les étapes suivantes :
- prélever un échantillon du fluide corporel,
- mettre en contact une première portion de l'échantillon avec une électrode sélective d'ions comprenant une électrode de référence interne (1, 2 ; 11, 12), une membrane sélective du carbonate en phase liquide (3 ; 13) et une membrane de discrimination (4 ; 14) comprenant une membrane hydrophile possédant une taille moyenne de pore inférieure à 0,1 µm, ladite membrane sélective du carbonate en phase liquide (3 ; 13) étant placée en contact intime avec ladite membrane de discrimination (4 ; 14),
- mettre en contact une seconde portion de l'échantillon, qui est en contact avec la première portion de l'échantillon, avec une électrode de référence externe,
- mesurer la différence de tension entre l'électrode de référence externe et l'électrode sélective d'ions correspondant à la concentration en ions carbonates dans l'échantillon, et
- comparer ladite tension à des tensions de référence correspondant à des concentrations prédéterminées en dioxyde de carbone de manière à déterminer la concentration du dioxyde de carbone se trouvant dans l'échantillon.

2. Procédé selon la revendication 1, dans lequel le pH de l'échantillon est mesuré simultanément.

3. Procédé selon la revendication 1 ou 2, dans lequel le fluide corporel comprend le sang, le plasma ou le sérum.

4. Procédé selon l'une quelconque des revendications précédentes, dans lequel une membrane de discrimination (4 ; 14) choisie dans le groupe constitué par les membranes faites de fibres de cellulose, de fibres de cellulose régénérée et de fibres de dérivés cellulosiques est utilisée.

5. Procédé selon l'une quelconque des revendications précédentes, dans lequel une membrane de discrimination (4 ; 14) possédant une épaisseur inférieure à 200 µm est utilisée.

6. Procédé selon l'une quelconque des revendications précédentes, dans lequel une membrane de discrimination (4 ; 14) comprenant une membrane faite de fibres de viscose et possédant une taille moyenne de pore inférieure à environ 0,01 µm est utilisée.

7. Procédé selon la revendication 6, dans lequel une membrane de discrimination (4 ; 14) comprenant un film de cellophane possédant une épaisseur inférieure à environ 50 µm est utilisée.

8. Procédé selon l'une quelconque des revendications précédentes, dans lequel une membrane sélective du carbonate (3 ; 13) essentiellement exempte de polymères dissous dans la phase liquide est utilisée.

9. Procédé selon l'une quelconque des revendications précédentes, dans lequel une membrane sélective du carbonate (3 ; 13) comprenant une substance se liant spécifiquement aux ions carbonates et un solvant est utilisée.

10. Procédé selon l'une quelconque des revendications précédentes, dans lequel une membrane sélective du carbonate (3 ; 13) comprenant :
- une substance se liant spécifiquement aux ions carbonates,
- une substance médiatrice, telle qu'un sel d'ammonium quaternaire, choisie de préférence dans le groupe constitué par le chlorure de tridodécylméthylammonium et le carbonate de tridodécylméthylammonium,
- une substance de blocage, telle qu'un sel d'ammonium quaternaire, de préférence le tétrakis(4-chlorophényl)borate de tétradodécylammonium, et
- un solvant
est utilisée.

11. Procédé selon la revendication 9 ou 10, dans lequel ladite substance se liant spécifiquement aux ions carbonates est un ionophore, choisi de préférence dans le groupe constitué par la 4-butyl-α,α,α-trifluoroacétophénone, la 4-hexadécyl-α,α,α-trifluoroacétophénone, le 1-(dodécylsulfonyl)-4-trifluoroacétylbenzène, le 4-heptyl-α,α,α-trifluoroacétylbenzoate, le 4-N-dodécyl-α,α,α-trifluoroacétylacétanilide.

12. Procédé selon la revendication 9 ou 10, dans lequel ledit solvant est un adipate, un sébacate, un éther ou un de leurs mélanges, choisi de préférence dans le groupe constitué par l'adipate de bis(2-éthylhexyle), le sébacate de bis(2-éthylhexyle) et le 2-nitrophényl octyl éther.

13. Procédé selon l'une quelconque des revendications précédentes, dans lequel une électrode de référence interne (1, 2 ; 11, 12) au moins partiellement immergée dans la membrane sélective du carbonate (3 ; 13) est utilisée.

14. Utilisation d'une électrode sélective d'ions, comprenant :
- une électrode de référence interne (1, 2 ; 11, 12),
- une membrane sélective du carbonate en phase liquide (3 ; 13), et
- une membrane de discrimination (4 ; 14) comprenant une membrane hydrophile possédant une taille moyenne de pore inférieure à 0,1 µm, ladite membrane sélective d'ions en phase liquide (3 ; 13) étant placée en contact intime avec ladite membrane de discrimination (4 ; 14) pour déterminer le dioxyde de carbone dans des fluides corporels.

15. Utilisation selon la revendication 14, dans laquelle ladite membrane de discrimination (4 ; 14) est choisie dans le groupe constitué par les membranes faites de fibres de cellulose, de fibres de cellulose régénérée et de fibres de dérivés cellulosiques.

16. Utilisation selon la revendication 14 ou 15, dans laquelle l'épaisseur de ladite membrane de discrimination (4 ; 14) est inférieure à 200 µm.

17. Utilisation selon l'une quelconque des revendications 14 à 16, dans laquelle ladite membrane de discrimination (4 ; 14) comprend une membrane faite de fibres de viscose et possède une taille moyenne de pore inférieure à environ 0,01 µm.

18. Utilisation selon la revendication 17, dans laquelle ladite membrane de discrimination (4 ; 14) comprend un film de cellophane possédant une épaisseur inférieure à environ 50 µm.

19. Utilisation selon l'une quelconque des revendications 14 à 18, dans laquelle ladite membrane sélective du carbonate (3 ; 13) est essentiellement exempte de polymères dissous dans la phase liquide.

20. Utilisation selon l'une quelconque des revendications 14 à 19, dans laquelle ladite membrane sélective du carbonate (3 ; 13) comprend une substance se liant spécifiquement aux ions carbonates et un solvant.

21. Utilisation selon l'une quelconque des revendications 14 à 20, dans laquelle ladite membrane sélective du carbonate (3 ; 13) comprend une substance se liant spécifiquement aux ions carbonates, une substance médiatrice, une substance de blocage et un solvant.

22. Utilisation selon la revendication 20 ou 21, dans laquelle ladite substance se liant spécifiquement aux ions carbonates est un ionophore.

23. Utilisation selon la revendication 22, dans laquelle ledit ionophore est choisi dans le groupe constitué par la 4-butyl-α,α,α-trifluoroacétophénone, la 4-hexadécyl-α,α,α-trifluoroacétophénone, le 1-(dodécylsulfonyl)-4-trifluoroacétylbenzène, le 4-heptyl-α,α,α-trifluoroacétylbenzoate, le 4-N-dodécyl-α,α,α-trifluoroacétylacétanilide.

24. Utilisation selon la revendication 21, dans laquelle ladite substance médiatrice est un sel d'ammonium quaternaire.

25. Utilisation selon la revendication 24, dans laquelle ladite substance médiatrice est un sel d'ammonium quaternaire choisi dans le groupe constitué par le chlorure de tridodécylméthylammonium et le carbonate de tridodécylméthylammonium.

26. Utilisation selon la revendication 21, dans laquelle ladite substance de blocage est un sel d'ammonium quaternaire.

27. Utilisation selon la revendication 26, dans laquelle ledit sel d'ammonium quaternaire est le tétrakis(4-chlorophényl)borate de tétradodécylammonium.

28. Utilisation selon la revendication 20 ou 21, dans laquelle ledit solvant est un adipate, un sébacate, un éther ou un de leurs mélanges.

29. Utilisation selon la revendication 28, dans laquelle ledit solvant est choisi dans le groupe constitué par l'adipate de bis(2-éthylhexyle), le sébacate de bis(2-éthylhexyle) et le 2-nitrophényl octyl éther.

30. Utilisation selon l'une quelconque des revendications 14 à 29, dans laquelle l'électrode de référence interne (1, 2 ; 11, 12) est au moins partiellement immergée dans la membrane sélective du carbonate (3 ; 13).
